# EUROPÄISCHE PATENTSCHRIFT

(11) **EP 3 646 894 B1**
(45) Veröffentlichungstag und Bekanntmachung des Hinweises auf die Patenterteilung: **10.05.2023**
(21) Anmeldenummer: 19205546.5
(22) Anmeldetag: 28.10.2019
(51) Int. Cl.: A61L 27/04, A61L 27/30, A61L 27/56, A61L 27/32

(54) **IMPLANTAT, VORZUGSWEISE ZUM BEHANDELN EINES KNOCHEN- UND/ODER KNORPELDEFEKTS**
IMPLANT, PREFERABLY FOR TREATING A BONE AND / OR CARTILAGE DEFECT
IMPLANT, DE PRÉFÉRENCE DESTINÉ AU TRAITEMENT D'UN DÉFAUT OSSEUX ET / OU DE CARTILAGE

(30) Priorität: 29.10.2018 DE 102018218498
(43) Veröffentlichungstag der Anmeldung: 06.05.2020
(73) Patentinhaber: Aesculap AG, 78532 Tuttlingen (DE)
(72) Erfinder: Mulliez, Marie Anne, 78532 Tuttlingen (DE)
(74) Vertreter: Patentanwälte Ruff, Wilhelm, Beier, Dauster & Partner mbB

(56) Entgegenhaltungen:
- WO-A1-2009/115616
- US-A1- 2007 225 822
- US-A1- 2012 109 331

## Beschreibung

### ANWENDUNGSGEBIET UND STAND DER TECHNIK

Die Erfindung betrifft ein Implantat, vorzugsweise zum Behandeln eines Knochen- und/oder Knorpeldefekts.

Zum Behandeln von Knochendefekten kommen insbesondere Pyrocarbonimplantate zum Einsatz. Pyrocarbonimplantate weisen üblicherweise ein Graphitsubstrat auf, welches mit pyrolytischem Kohlenstoff beschichtet ist. Zum Behandeln eines Knochendefekts muss ein solches Pyrocarbonimplantat am Knochen befestigt werden.

Aus der WO 2009/115616 A1 ist ein Pyrocarbonimplantat zum Behandeln eines Gelenkknochendefekts bekannt, welches mittels einer Klebepolymer- oder Elastomerschicht am Knochen eines Patienten befestigt wird.

Gegenstand der WO 2009/115613 A1 ist ein Pyrocarbonimplantat zum Behandeln eines Knorpeldefekts, bei welchem die Befestigung im Körper eines Patienten mittels Knochenanker realisiert wird.

Die US 2012/0109331 A1 offenbart ein Implantat zum Behandeln eines Azetabulumdefekts mit einer porösen Knochenanwachseinrichtung und einer konkav gestalteten Artikulationsoberfläche.

Die US 2007/0225822 A1 offenbart ein Implantat mit einer Pyrocarbonschicht und einer Metallschicht, wobei die Pyrocarbonschicht eine konvexe Außenfläche des Implantats und die Metallschicht eine konkave Außenfläche des Implantats bildet.

Nachteilig bei den aus dem Stand der Technik bekannten Implantaten ist insbesondere der oftmals hohe Knochenverbrauch. Der hohe Knochenverbrauch rührt in aller Regel daher, dass ungeachtet der Größe und Lokalisation des Knorpeldefektes eine Totalendoprothese (TEP) implantiert wird. Bei der Behandlung von Gelenkknorpeldefekten bedeutet dies, dass die natürlichen Knorpelgleitlager vollständig ersetzt werden, woraus ein höherer Knochenverbrauch als erforderlich resultiert.

### AUFGABE UND LÖSUNG

Aufgabe der Erfindung ist es, ein Implantat bereitzustellen, welches sich vor allem zum Behandeln eines Knochen- und/oder Knorpeldefekts eignet und insbesondere wenigstens einen Teil der bei gattungsgemäßen Implantaten auftretenden Nachteile vermeidet. Das Implantat soll insbesondere eine knochenschonende Versorgung von Knochen- und/oder Knorpeldefekten ermöglichen.

Diese Aufgabe wird gelöst durch ein Implantat mit den Merkmalen gemäß unabhängigem Anspruch 1. Bevorzugte Ausgestaltungen des Implantats sind Gegenstand der abhängigen Ansprüche 2 bis 14 sowie der vorliegenden Beschreibung. Der Wortlaut sämtlicher Ansprüche wird hiermit durch ausdrückliche Bezugnahme zum Inhalt der vorliegenden Beschreibung gemacht.

Die Erfindung betrifft ein Implantat, vorzugsweise zum Behandeln, insbesondere zum Auskleiden und/oder Unterfüttern und/oder wenigstens teilweisen Auffüllen und/oder Abdichten und/oder biologischen Rekonstruieren und/oder Ersatz, insbesondere Totalersatz oder Teilersatz, eines Knochendefekts und/oder Knorpeldefekts. Bei dem Implantat handelt es sich vorzugsweise um ein chirurgisches Implantat.

Das Implantat weist eine Pyrocarbonschicht und eine Knochenanwachseinrichtung auf.

Der Ausdruck "Knochendefekt" bedeutet im Sinne der vorliegenden Erfindung einen durch Verlust oder Beschädigung von Knochengewebe, insbesondere Gelenksknochengewebe, vorzugsweise Hüftgelenks- oder Kniegelenksknochengewebe, oder von Wirbelkörpergewebe betroffenen Knochenbereich, insbesondere Gelenksknochenbereich, vorzugsweise Hüftgelenks- oder Kniegelenksknochenbereich, oder Wirbelkörperbereich. Der Knochenverlust oder die Knochenbeschädigung kann die Folge einer Knochenfraktur, eines Knochentraumas, einer Nekrose, einer Ausdünnung (beispielsweise infolge von Osteoporose), einer Knochenerkrankung wie Tumorerkrankung, einer fehlenden mechanischen Belastung (sogenanntes Stress Shielding) oder die Folge einer chirurgischen Intervention/Reintervention, insbesondere einer Revision nach einem vollständigen Knie- oder Hüftgelenkersatz, sein.

Insbesondere kann der Ausdruck "Knochendefekt" im Sinne der vorliegenden Erfindung einen periprothetischen Knochendefekt, d.h. einen durch periprothetischen Knochengewebsverlust, insbesondere aufgrund von mechanischer Überlastung und/oder Abrieb-induzierter Osteolyse und/oder Implantatmigration, betroffenen Knochenbereich bedeuten.

Vorzugsweise handelt es sich bei dem Knochendefekt um einen Gelenksknochendefekt, insbesondere Kniegelenksknochendefekt oder Hüftgelenksknochendefekt, bevorzugt Azetabul umdefekt.

Die Pyrocarbonschicht bildet eine konkave Außenfläche oder eine konkave Außenschicht des Implantats und die Knochenanwachseinrichtung eine konvexe Außenfläche oder eine konvexe Außenschicht des Implantats. Dies ist besonders vorteilhaft im Hinblick auf die Behandlung eines Azetabulumdefekts.

Grundsätzlich kann die Pyrocarbonschicht nur teilweise, d.h. nur abschnitts- oder bereichsweise, eine konkave Außenfläche oder Außenschicht des Implantats oder eine durchgehende, d.h. unterbrechungsfreie, konkave Außenfläche oder Außenschicht des Implantats bilden.

Ferner kann grundsätzlich die Knochenanwachseinrichtung nur teilweise, d.h. nur abschnitts- oder bereichsweise, eine konvexe Außenfläche oder Außenschicht des Implantats oder eine durchgehende, d.h. unterbrechungsfreie, konvexe Außenfläche oder Außenschicht des Implantats bilden.

Vorzugsweise bildet die Pyrocarbonschicht eine durchgehende, d.h. unterbrechungsfreie, konkave Außenfläche oder Außenschicht des Implantats und die Knochenanwachseinrichtung eine durchgehende, d.h. unterbrechungsfreie, konvexe Außenfläche oder Außenschicht des Implantats.

Alternativ bevorzugt bildet die Pyrocarbonschicht eine durchgehende, d.h. unterbrechungsfreie, konkave Außenfläche oder Außenschicht des Implantats und die Knochenanwachseinrichtung nur teilweise, d.h. nur abschnitts- oder bereichsweise, eine konvexe Außenfläche oder Außenschicht des Implantats.

Der Ausdruck "Knorpeldefekt" bedeutet im Sinne der vorliegenden Erfindung einen durch Verlust oder Beschädigung von Knorpelgewebe, insbesondere Gelenksknorpelgewebe, vorzugsweise Hüftgelenks- oder Kniegelenksknorpelgewebe, oder Meniskus, betroffenen Knorpelbereich, insbesondere Gelenksknorpelbereich, vorzugsweise Hüftgelenks- oder Kniegelenksknorpelbereich oder Meniskusbereich. Der Knorpelverlust kann beispielsweise die Folge eines Knorpeltraumas oder einer Knorpelerkrankung sein.

Weiterhin kann der Ausdruck "Knochendefekt" im Sinne der vorliegenden Erfindung einen menschlichen Knochendefekt oder einen tierischen Knochendefekt bedeuten.

Entsprechend kann der Ausdruck "Knorpeldefekt" im Sinne der vorliegenden Erfindung einen menschlichen Knorpeldefekt oder einen tierischen Knorpeldefekt bedeuten.

Unter dem Ausdruck "tierischer Knochendefekt" soll im Sinne der vorliegenden Erfindung ein Knochendefekt eines nicht humanen Säugetiers, wie beispielsweise eines Pferds, einer Kuh, einer Ziege, eines Schafs, eines Schweins oder eines Nagetiers, wie beispielsweise eines Hasen, einer Ratte oder einer Maus, verstanden werden.

Entsprechend soll unter dem Ausdruck "tierischer Knorpeldefekt" im Sinne der vorliegenden Erfindung ein Knorpeldefekt eines nicht humanen Säugetiers, wie beispielsweise eines Pferds, einer Kuh, einer Ziege, eines Schafs, eines Schweins oder eines Nagetiers, wie beispielsweise eines Hasen, einer Ratte oder einer Maus, verstanden werden.

Unter dem Ausdruck "Pyrocarbonschicht" soll im Sinne der vorliegenden Erfindung eine Schicht verstanden werden, welche Pyrocarbon aufweist oder aus Pyrocarbon besteht.

Beispielsweise kann die Pyrocarbonschicht einen Anteil an Pyrocarbon > 50 Gew.-%, insbesondere > 80 Gew.-%, bevorzugt > 90 Gew.-%, weiter bevorzugt > 95 Gew.-%, besonders bevorzugt > 98 Gew.-%, aufweisen, bezogen auf das Gesamtgewicht der Pyrocarbonschicht. Insbesondere kann die Pyrocarbonschicht einen Anteil an Pyrocarbon von 55 Gew.-% bis 100 Gew.-%, insbesondere 80 Gew.-% bis 99,5 Gew.-%, bevorzugt 90 Gew.-% bis 99 Gew.-%, aufweisen, bezogen auf das Gesamtgewicht der Pyrocarbonschicht.

Unter dem Ausdruck "Pyrocarbon" soll im Sinne der vorliegenden Erfindung pyrolytischer Kohlenstoff verstanden werden, welcher auch als Pyrokohlenstoff bezeichnet werden kann. Hierbei handelt es sich um eine feste Form des Kohlenstoffs, welche bei der Pyrolyse eines gasförmigen oder flüssigen Kohlenwasserstoffs, insbesondere bei einer Temperatur von 700 °C bis 2200 °C, auf die Oberfläche eines Substrats oder Trägers, wie insbesondere Graphit, abgeschieden wird. Der pyrolytische Kohlenstoff bzw. Pyrokohlenstoff weist nahezu ausschließlich sp²-hybridisierte Kohlenstoffatome auf. Die Nahordnung von pyrolytischem Kohlenstoff ähnelt der Nahordnung von Graphit. Pyrolytischer Kohlenstoff weist bevorzugt eine sogenannte turbostratische Mikrostruktur auf. Dies bedeutet, dass der pyrolytische Kohlenstoff auf der Nanometerskala bevorzugt Bereiche aufweist oder aus Bereichen besteht, in welchen Graphenebenen oder graphenähnliche Ebenen nahezu parallel und äquidistant liegen.

Alternativ oder in Kombination können diese Ebenen sowohl gegeneinander verdreht als auch verschoben sein und obendrein Fehlstellen oder Welligkeiten aufweisen.

Unter dem Ausdruck "Knochenanwachseinrichtung" soll im Sinne der vorliegenden Erfindung eine Einrichtung, d.h. ein Element, eine Komponente oder eine Struktur, des Implantats verstanden werden, welche zum Anwachsen von Knochen auf einer Oberfläche des Implantats (sogenannte Knochenapposition) und/oder zum Einwachsen von Knochen in das Implantat hinein, d.h. zum Einwachsen von Knochen in das Innere des Implantats, und/oder zum Ersatz von Knorpel ausgebildet ist.

Durch die Erfindung wird mit besonderem Vorteil ein pyrocarbonhaltiges Implantat bereitgestellt, welches insbesondere für eine knochen- und/oder knorpelschonende Versorgung von Knochen- und/oder Knorpeldefekten gestaltet ist. Hierbei werden zum einen die besonderen mechanischen und tribologischen Eigenschaften von Pyrocarbon zur Geltung gebracht, welches direkt mit einem natürlichen Gewebe, insbesondere Knochen und/oder Knorpel, gepaart werden oder artikulieren kann. Vorzugsweise weist die Pyrocarbonschicht des Implantats hierzu eine Oberfläche, insbesondere eine glatte und/oder porenfreie Oberfläche, auf, welche zum Anlegen oder Auflegen eines Knochens und/oder Knorpels, insbesondere zum Artikulieren oder Gleiten mit einem Knochen und/oder Knorpel, ausgebildet ist. Anders ausgedrückt, weist die Pyrocarbonschicht des Implantats vorzugsweise eine Anlege- oder Auflagefläche, insbesondere eine glatte und/oder porenfreie Anlege- oder Auflagefläche, für einen Knochen und/oder einen Knorpel auf. Die Anlege- oder Auflagefläche kann auch als Artikulations- oder Gleitfläche für einen Knochen und/oder einen Knorpel bezeichnet werden.

Zum anderen ist durch die erfindungsgemäß vorgesehene Knochenanwachseinrichtung eine Befestigung, vorzugsweise eine knochen- und/oder knorpelschonende Befestigung, des Implantats im Körper eines Patienten erzielbar. Hierfür eignen sich insbesondere die im Folgenden im Zusammenhang der Knochenanwachseinrichtung beschriebenen Materialien.

Eine knochen- und/oder knorpelschonende Befestigung des Implantats erlaubt wiederum mit besonderem Vorteil eine knochen- und/oder knorpelschonende Versorgung von Knochen- und/oder Knorpeldefekten, insbesondere Gelenkknochen- und/oder Gelenkknorpeldefekten.

Insbesondere eröffnet das erfindungsgemäße Implantat knochenschonende Versorgungsmöglichkeiten im Knie- oder Hüftgelenk. Dadurch kann das Risiko eines vollständigen Knochengelenk- und/oder Knorpelgelenkersatzes minimiert werden. Durch das Implantat können in vorteilhafter Weise insbesondere sowohl kleine Gelenkknochen- und/oder Gelenkknorpeldefekte als auch einseitige Artikulationsdefekte versorgt werden, ohne dass das ganze Gelenk ersetzt werden muss.

Zweckmäßigerweise sind die Pyrocarbonschicht und die Knochenanwachseinrichtung unterschiedlich ausgebildet oder gestaltet. Anders ausgedrückt, weist das Implantat zweckmäßigerweise eine Pyrocarbonschicht und eine zu der Pyrocarbonschicht unterschiedliche Knochenanwachseinrichtung auf.

Insbesondere ist die Knochenanwachseinrichtung frei von Pyrocarbon, d.h. frei von pyrolytischem Kohlenstoff bzw. Pyrokohlenstoff.

Bevorzugt weist die Pyrocarbonschicht eine Außenfläche auf, welche zu natürlichen Gelenkflächen komplementär ist. Die Außenfläche ist konkav gestaltet.

Vorzugsweise ist die Pyrocarbonschicht schalenförmig oder kugelschalensegmentförmig, insbesondere halbkugelschalenförmig, gestaltet.

Grundsätzlich kann die Pyrocarbonschicht aber auch andere Formen und/oder Geometrien aufweisen. Beispielsweise kann die Pyrocarbonschicht eine Außenfläche aufweisen, welche komplementär zu einem Femurkopf, einem Azetabulum, einem Tibiaplateau, einer Trochlea, einer Bandscheibe, einer Kondyle, einer Patella oder einem Meniskus ist. Die Pyrocarbonschicht kann beispielsweise sichelförmig, keilförmig oder platten- oder scheibenförmig, insbesondere rechteckscheibenförmig, quadratscheibenförmig, kreisscheibenförmig, halbkreisscheibenförmig, elliptisch oder ovalscheibenförmig, gestaltet sein.

Die Pyrocarbonschicht kann weiterhin eine Schichtdicke von 0,1 mm bis 5 mm, insbesondere 0,2 mm bis 2 mm, vorzugsweise 0,5 mm bis 1 mm, aufweisen.

In Ausgestaltung der Erfindung ist die Pyrocarbonschicht auf einem Pyrocarbonschichtträger ausgebildet, insbesondere aufgebracht. Insbesondere kann die Pyrocarbonschicht auf einer konkaven Fläche, insbesondere konkaven Außenfläche, oder auf einer konkaven Schicht, insbesondere konkaven Außenschicht, oder auf einer konvexen Fläche, insbesondere konvexen Außenfläche, oder auf einer konvexen Schicht, insbesondere konvexen Außenschicht, des Pyrocarbonschichtträgers ausgebildet, insbesondere aufgebracht, sein. Vorzugsweise ist die Pyrocarbonschicht unmittelbar auf dem Pyrocarbonschichtträger, insbesondere auf einer konkaven Fläche, insbesondere konkaven Außenfläche, oder auf einer konkaven Schicht, insbesondere konkaven Außenschicht, oder auf einer konvexen Fläche, insbesondere konvexen Außenfläche, oder auf einer konvexen Schicht, insbesondere konvexen Außenschicht, des Pyrocarbonschichtträgers ausgebildet, insbesondere aufgebracht.

Unter dem Ausdruck "Pyrocarbonschichtträger" soll im Sinne der vorliegenden Erfindung ein Material verstanden werden, welches zum Trägern der Pyrocarbonschicht ausgebildet ist, d.h. als Träger für die Pyrocarbonschicht verwendet werden kann.

Der Pyrocarbonschichtträger ist vorzugsweise schalenförmig oder kugelschalensegmentförmig, insbesondere halbkugelschalenförmig, gestaltet.

Grundsätzlich kann der Pyrocarbonschichtträger aber auch andere Formen und/oder Geometrien aufweisen. Beispielsweise kann der Pyrocarbonschichtträger sichelförmig, keilförmig oder platten- oder scheibenförmig, insbesondere rechteckscheibenförmig, quadratscheibenförmig, kreisscheibenförmig, halbkreisscheibenförmig, elliptisch oder ovalscheibenförmig, gestaltet sein.

Des Weiteren kann der Pyrocarbonschichtträger eine der Knochenanwachseinrichtung zugewandte strukturierte und/oder offenporige Oberfläche aufweisen. Bei der Oberfläche handelt es sich vorzugsweise um eine Außenoberfläche des Pyrocarbonschichtträgers. Die Oberfläche des Pyrocarbonschichtträgers kann insbesondere Poren mit einem mittleren Porendurchmesser, bestimmt mittels Rasterelektronenmikroskopie und/oder Quecksilber-Porosimetrie, von 50 µm bis 1500 µm, bevorzugt 100 µm bis 1000 µm, besonders bevorzugt 100 µm bis 550 µm, aufweisen. Durch die in diesem Absatz beschriebenen Ausführungsformen kann eine Ausbildung, insbesondere Aufbringung, der Knochenanwachseinrichtung und/oder eines Metall- oder Legierungsformkörpers, insbesondere wie nachfolgend noch eingehender beschrieben werden wird, auf dem Pyrocarbonschichtträger erleichtert werden.

Der Pyrocarbonschichtträger kann weiterhin eine Dicke, insbesondere Schichtdicke, von 0,1 mm bis 10 mm, insbesondere 0,5 mm bis 5 mm, vorzugsweise 0,5 mm bis 1,5 mm, aufweisen.

In weiterer Ausgestaltung der Erfindung handelt es sich bei dem Pyrocarbonschichtträger um einen Graphitformkörper.

Unter dem Ausdruck "Graphitformkörper" soll im Sinne der vorliegenden Erfindung ein Formkörper verstanden werden, welcher Graphit aufweist oder aus Graphit besteht. Bevorzugt weist der Graphitformkörper einen Anteil an Graphit > 50 Gew.-%, insbesondere > 80 Gew.-%, bevorzugt > 90 Gew.-%, weiter bevorzugt > 95 Gew.-%, besonders bevorzugt > 98 Gew.-%, auf, bezogen auf das Gesamtgewicht des Graphitformkörpers. Beispielsweise kann der Graphitformkörper einen Anteil an Graphit von 55 Gew.-% bis 100 Gew.-%, insbesondere 80 Gew.-% bis 99,5 Gew.-%, bevorzugt 90 Gew.-% bis 99 Gew.-%, aufweisen, bezogen auf das Gesamtgewicht des Graphitformkörpers.

In weiterer Ausgestaltung der Erfindung ist die Knochenanwachseinrichtung wenigstens teilweise, d.h. wenigstens abschnitts- oder bereichsweise, insbesondere nur teilweise, d.h. nur abschnitts- oder bereichsweise, oder vollständig, d.h. durchgehend oder vollflächig, auf der Pyrocarbonschicht, insbesondere auf einer konkaven oder konvexen Fläche, insbesondere konkaven oder konvexen Außenfläche, oder auf einer konkaven oder konvexen Schicht, insbesondere konkaven oder konvexen Außenschicht, der Pyrocarbonschicht, oder auf dem Pyrocarbonschichtträger, insbesondere auf einer konkaven oder konvexen Fläche, insbesondere konkaven oder konvexen Außenfläche, oder auf einer konkaven oder konvexen Schicht, insbesondere konkaven oder konvexen Außenschicht, des Pyrocarbonschichtträgers, ausgebildet, insbesondere aufgebracht. Die Knochenanwachseinrichtung kann dabei insbesondere unmittelbar auf der Pyrocarbonschicht oder dem Pyrocarbonschichtträger ausgebildet, insbesondere aufgebracht, sein. Durch eine nur teilweise auf der Pyrocarbonschicht oder dem Pyrocarbonschichtträger ausgebildete, insbesondere aufgebrachte, Knochenanwachseinrichtung können in besonderer Weise sowohl die Elastizität als auch die Flexibilität der Pyrocarbonschicht und/oder des Pyrocarbonschichtträgers, insbesondere eines Verbundes aus der Pyrocarbonschicht und dem Pyrocarbonschichtträger, aufrechterhalten werden.

Vorzugsweise ist der Pyrocarbonschichtträger zwischen der Pyrocarbonschicht und der Knochenanwachseinrichtung angeordnet. Hierzu kann der Pyrocarbonschichtträger, insbesondere entlang einer gemeinsamen Grenzfläche, in direktem Kontakt mit der Pyrocarbonschicht und/oder, insbesondere entlang einer gemeinsamen Grenzfläche, in direktem Kontakt mit der Knochenanwachseinrichtung stehen. Besonders bevorzugt steht der Pyrocarbonschichtträger sowohl, insbesondere entlang einer gemeinsamen Grenzfläche, in direktem Kontakt mit der Pyrocarbonschicht als auch, insbesondere entlang einer gemeinsamen Grenzfläche, in direktem Kontakt mit der Knochenanwachseinrichtung.

In weiterer Ausgestaltung der Erfindung weist die Knochenanwachseinrichtung eine strukturierte, insbesondere aufgeraute, Oberfläche auf. Vorzugsweise weist die Knochenanwachseinrichtung eine Oberfläche mit Erhebungen und/oder Vertiefungen auf.

Dadurch kann mit besonderem Vorteil das Anwachsen von Knochen und/oder Knorpel auf einer Oberfläche des Implantats, insbesondere auf einer Außenoberfläche der Knochenanwachseinrichtung, gefördert und/oder stimuliert werden. Dies erleichtert eine sichere und insbesondere knochen- und/oder knorpelschonende Befestigung des Implantats im Körper eines Patienten.

Bevorzugt weist die Knochenanwachseinrichtung eine Außenfläche auf, welche komplementär zu einer natürlichen Gelenkfläche ist. Die Außenfläche ist konvex gestaltet.

Vorzugsweise ist die Knochenanwachseinrichtung schalenförmig oder kugelschalensegmentförmig, insbesondere halbkugelschalenförmig, gestaltet.

Grundsätzlich kann die Knochenanwachseinrichtung aber auch andere Formen und/oder Geometrien aufweisen. Beispielsweise kann die Knochenanwachseinrichtung eine Außenfläche aufweisen, welche komplementär zu einem Femurkopf, einem Azetabulum, einem Tibiaplateau, einer Trochlea, einer Bandscheibe, einer Kondyle, einer Patella oder einem Meniskus ist. Beispielsweise kann die Knochenanwachseinrichtung sichelförmig, keilförmig oder platten- oder scheibenförmig, insbesondere rechteckscheibenförmig, quadratscheibenförmig, kreisscheibenförmig, halbkreisscheibenförmig, elliptisch oder ovalscheibenförmig, gestaltet sein.

In weiterer Ausgestaltung der Erfindung ist die Knochenanwachseinrichtung, insbesondere eine Oberfläche, vorzugsweise Außenoberfläche, davon, offenporig gestaltet. Dadurch ist mit besonderem Vorteil sowohl ein Anwachsen von Knochen und/oder Knorpel auf einer Oberfläche, bevorzugt Außenoberfläche, der Knochenanwachseinrichtung als auch ein Einwachsen von Knochen und/oder Knorpel in die Knochenanwachseinrichtung hinein erzielbar. Dadurch wird eine besonders wirkungsvolle Möglichkeit geschaffen, das Implantat im Körper eines Patienten zu befestigen.

Insbesondere kann die Knochenanwachseinrichtung Poren mit einem mittleren Porendurchmesser, bestimmt mittels Rasterelektronenmikroskopie und/oder Quecksilber-Porosimetrie, von 50 µm bis 1500 µm, bevorzugt 100 µm bis 1000 µm, besonders bevorzugt 100 µm bis 550 µm, aufweisen.

Vorzugsweise weist die Knochenanwachseinrichtung interkonnektierende Poren und/oder Mikroporen, insbesondere mit einem mittleren Porendurchmesser, bestimmt mittels Rasterelektronenmikroskopie und/oder Quecksilber-Porosimetrie, von 0,001 µm bis 50 µm, bevorzugt 0,01 µm bis 30 µm, besonders bevorzugt 0,1 µm bis 20 µm, auf.

Weiterhin kann die Knochenanwachseinrichtung eine Porosität nach DIN EN ISO 993-1 von 30% bis 90% aufweisen.

In weiterer Ausgestaltung der Erfindung ist die Knochenanwachseinrichtung schichtförmig, d.h. als Schicht oder Beschichtung, insbesondere einschichtförmig, d.h. in Form einer einzigen Schicht oder einer einzigen Beschichtung, oder mehrschichtförmig, d.h. in Form einer Vielzahl von Schichten oder Beschichtungen, gestaltet. Dies hat den Vorteil, dass eine Beeinträchtigung der mechanischen, insbesondere flexiblen oder elastischen, Eigenschaften der Pyrocarbonschicht und/oder des Pyrocarbonschichtträgers, insbesondere eines Verbundes aus der Pyrocarbonschicht und dem Pyrocarbonschichtträger, über eine entsprechende Schichtdicke der Knochenanwachseinrichtung vermieden werden kann. Erfindungsgemäß ist es daher besonders bevorzugt, wenn die Knochenanwachseinrichtung derart dünn gestaltet ist, dass einerseits die mechanischen Eigenschaften, insbesondere die Flexibilität oder Elastizität, der Pyrocarbonschicht und/oder des Pyrocarbonschichtträgers, insbesondere eines Verbundes aus der Pyrocarbonschicht und dem Pyrocarbonschichtträger, nicht oder wenigstens nicht nennenswert beeinträchtigt wird und andererseits die Schichtdicke der Knochenanwachseinrichtung ausreichend dick ist, um eine ausreichende Primärstabilität (sogenanntes Pressfit) zu gewährleisten.

In weiterer Ausgestaltung der Erfindung weist die Knochenanwachseinrichtung eine Schichtdicke von 5 µm bis 50 µm, insbesondere 5 µm bis 40 µm, bevorzugt 5 µm bis 30 µm, auf. Die in diesem Absatz beschriebenen Schichtdicken haben den Vorteil, dass sie insbesondere im Falle einer calciumhaltigen Knochenanwachseinrichtung, d.h. einer Knochenanwachseinrichtung, welche ein calciumhaltiges Material aufweist oder aus einem solchen Material besteht, zu keiner (nennenswerten) Beeinträchtigung der mechanischen Eigenschaften, insbesondere der Flexibilität oder Elastizität, der Pyrocarbonschicht und/oder des Pyrocarbonschichtträgers, insbesondere eines Verbundes aus der Pyrocarbonschicht und dem Pyrocarbonschichtträger, führen und trotzdem dick genug sind, um eine ausreichende Primärstabilität (sogenanntes Pressfit) zu gewährleisten.

In weiterer Ausgestaltung der Erfindung weist die Knochenanwachseinrichtung eine Schichtdicke von 250 µm bis 700 µm, insbesondere 300 µm bis 400 µm, bevorzugt 350 µm, auf. Die in diesem Absatz beschriebenen Schichtdicken haben den Vorteil, das sie insbesondere im Falle einer metall- oder legierungshaltigen oder einer aus einem Metall oder einer Legierung bestehenden Knochenanwachseinrichtung zu keiner (nennenswerten) Beeinträchtigung der mechanischen Eigenschaften, insbesondere der Flexibilität oder Elastizität, der Pyrocarbonschicht und/oder des Pyrocarbonschichtträgers, insbesondere eines Verbundes aus der Pyrocarbonschicht und dem Pyrocarbonschichtträger, führen und trotzdem dick genug sind, um eine ausreichende Primärstabilität (sogenanntes Pressfit) zu gewährleisten. Die Primärstabilität wird durch die Implantatgeometrie oder einen Übermaß gewährt. Die Sekundärstabilität wird dagegen durch das Knochenanwachsen gewährleistet.

Das Implantat weist ferner einen Metall- oder Legierungsformkörper auf.

Unter dem Ausdruck "Metallformkörper" soll im Sinne der vorliegenden Erfindung ein Formkörper verstanden werden, welcher ein Metall aufweist oder aus einem Metall besteht. Bevorzugt weist der Metallformkörper einen Anteil an Metall > 50 Gew.-%, insbesondere > 80 Gew.-%, bevorzugt > 90 Gew.-%, weiter bevorzugt > 95 Gew.-%, besonders bevorzugt > 98 Gew.-%, auf, bezogen auf das Gesamtgewicht des Metallformkörpers. Beispielsweise kann der Metallformkörper einen Anteil an Metall von 55 Gew.-% bis 100 Gew.-%, insbesondere 80 Gew.-% bis 99,5 Gew.-%, bevorzugt 90 Gew.-% bis 99 Gew.-%, aufweisen, bezogen auf das Gesamtgewicht des Metallformkörpers.

Entsprechend soll unter dem Ausdruck "Legierungsformkörper" im Sinne der vorliegenden Erfindung ein Formkörper verstanden werden, welcher eine Legierung aufweist oder aus einer Legierung besteht. Bevorzugt weist der Legierungsformkörper einen Anteil an Legierung > 50 Gew.-%, insbesondere > 80 Gew.-%, bevorzugt > 90 Gew.-%, weiter bevorzugt > 95 Gew.-%, besonders bevorzugt > 98 Gew.-%, auf, bezogen auf das Gesamtgewicht des Legierungsformkörpers. Beispielsweise kann der Legierungsformkörper einen Anteil an Metall von 55 Gew.-% bis 100 Gew.-%, insbesondere 80 Gew.-% bis 99,5 Gew.-%, bevorzugt 90 Gew.-% bis 99 Gew.-%, aufweisen, bezogen auf das Gesamtgewicht des Legierungsformkörpers.

Der Metall- oder Legierungsformkörper ist vorzugsweise schalenförmig oder kugelschalensegmentförmig, insbesondere halbkugelschalenförmig, gestaltet.

Grundsätzlich kann der Metall- oder Legierungsformkörper aber auch andere Formen und/oder Geometrien aufweisen. Beispielsweise kann der Metall- oder Legierungsformkörper sichelförmig, keilförmig oder platten- oder scheibenförmig, insbesondere rechteckscheibenförmig, quadratscheibenförmig, kreisscheibenförmig, halbkreisscheibenförmig, elliptisch oder ovalscheibenförmig, gestaltet sein.

In weiterer Ausgestaltung der Erfindung ist die Knochenanwachseinrichtung wenigstens teilweise, d.h. wenigstens abschnitts- oder bereichsweise, insbesondere nur teilweise, d.h. nur abschnittsweise oder bereichsweise, oder vollständig, d.h. durchgehend oder vollflächig, auf dem Metall- oder Legierungsformkörper, insbesondere auf einer konkaven oder konvexen Fläche, insbesondere konkaven oder konvexen Außenfläche, oder auf einer konkaven oder konvexen Schicht, insbesondere konkaven oder konvexen Außenschicht, des Metall- oder Legierungsformkörpers, ausgebildet, insbesondere aufgebracht. Die Knochenanwachseinrichtung kann dabei insbesondere unmittelbar auf dem Metall- oder Legierungsformkörper ausgebildet, insbesondere aufgebracht, sein. Durch eine nur teilweise auf dem Metall- oder Legierungsformkörper ausgebildete, insbesondere aufgebrachte, Knochenanwachseinrichtung können mit besonderem Vorteil auch kleinere Knochen- und/oder Knorpeldefekte, insbesondere kleinere Gelenkknochen- und/oder Gelenkknorpeldefekte, versorgt werden, ohne dass ein vollständiger Ersatz des betroffenen Knochens und/oder Knorpels, insbesondere des betroffenen Gelenkknochens und/oder Gelenkknorpels, erforderlich ist.

Der Metall- oder Legierungsformkörper kann weiterhin eine Dicke, insbesondere Schichtdicke, von 0,5 mm bis 10 mm, insbesondere 1 mm bis 5 mm, vorzugsweise 1 mm bis 2 mm, aufweisen.

Der Metall- oder Legierungsformkörper ist zwischen der Pyrocarbonschicht und der Knochenanwachseinrichtung angeordnet. Hierzu kann der Metall- oder Legierungsformkörper, insbesondere entlang einer gemeinsamen Grenzfläche, in direktem Kontakt mit der Pyrocarbonschicht und/oder, insbesondere entlang einer gemeinsamen Grenzfläche, in direktem Kontakt mit der Knochenanwachseinrichtung stehen. Besonders bevorzugt steht der Metall- oder Legierungsformkörper sowohl, insbesondere entlang einer gemeinsamen Grenzfläche, in direktem Kontakt mit der Pyrocarbonschicht als auch, insbesondere entlang einer gemeinsamen Grenzfläche, in direktem Kontakt mit der Knochenanwachseinrichtung.

Der Metall- oder Legierungsformkörper ist zwischen dem Pyrocarbonschichtträger und der Knochenanwachseinrichtung angeordnet. Hierzu kann der Metall- oder Legierungsformkörper, insbesondere entlang einer gemeinsamen Grenzfläche, in direktem Kontakt mit dem Pyrocarbonschichtträger und/oder, insbesondere entlang einer gemeinsamen Grenzfläche, in direktem Kontakt mit der Knochenanwachseinrichtung stehen. Besonders bevorzugt steht der Metall- oder Legierungsformkörper sowohl, insbesondere entlang einer gemeinsamen Grenzfläche, in direktem Kontakt mit dem Pyrocarbonschichtträger als auch, insbesondere entlang einer gemeinsamen Grenzfläche, in direktem Kontakt mit der Knochenanwachseinrichtung.

In weiterer Ausgestaltung der Erfindung handelt es sich bei der Knochenanwachseinrichtung um einen Metall- oder Legierungsformkörper.

In weiterer Ausgestaltung der Erfindung weist der Metall- oder Legierungsformkörper eine Dicke von 0,2 mm bis 2 mm, insbesondere 0,5 mm, auf. Die in diesem Absatz beschriebene Dicke des Metall- oder Legierungsformkörpers hat insbesondere den Vorteil, dass sie einerseits zu keiner (nennenswerten) Beeinträchtigung der mechanischen Eigenschaften, insbesondere der Flexibilität oder Elastizität, der Pyrocarbonschicht und/oder des Pyrocarbonschichtträgers, insbesondere eines Verbundes aus der Pyrocarbonschicht und dem Pyrocarbonschichtträger, führt und andererseits ausreichend ist, um eine unter Patientensicherheitsgesichtspunkten zufriedenstellende Primärstabilität (sogenanntes Pressfit) zu gewährleisten.

In weiterer Ausgestaltung der Erfindung handelt es sich bei der Knochenanwachseinrichtung um eine Keramik.

In weiterer Ausgestaltung der Erfindung weist die Knochenanwachseinrichtung ein calciumhaltiges Material auf oder besteht aus einem calciumhaltigen Material.

Das calciumhaltige Material kann grundsätzlich ausgewählt sein aus der Gruppe bestehend aus Calciumphosphate, Calciumsilikate, Calciumsulfate, Calciumcarbonate, Calciumoxide, Calciumhydroxide und Mischungen davon.

Das calciumhaltige Material kann insbesondere ausgewählt sein aus der Gruppe bestehend aus Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), α-Tricalciumphosphat (α-TCP), β-Tricalciumphosphat (β-TCP), amorphes Calciumphosphat (ACP), Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Fluorapatit, Chlorapatit, Carbonat-Fluor-Apatit, biphasisches Calciumphosphat, Tetracalciumphosphat (TTCP), Calciumsulfat (CaSO₄), Calciumsulfat-Hemihydrat (CaSO₄ × 0.5 H₂O), Calciumsulfat-Dihydrat (CaSO₄ × 2 H₂O), Calciumoxid (CaO), Calciumhydroxid (Ca(OH)₂), Calciumcarbonat (CaCO₃), Calciumglycerophosphat, Calciumcitrat, Calciumlactat, Calciumacetat, Calciumtartrat, Calciumchlorid (CaCl₂), Calciumsilicate und Mischungen davon.

Bevorzugt ist das calciumhaltige Material ein Calciumphosphat-Material, d.h. ein Material, welches Calciumphosphat aufweist oder aus Calciumphosphat besteht. Vorzugsweise ist das Calciumphosphat-Material ausgewählt aus der Gruppe bestehend aus Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), α-Tricalciumphosphat (α-TCP), β-Tricalciumphosphat (β-TCP), amorphes Calciumphosphat (ACP), Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Fluorapatit, Chlorapatit, Carbonat-Fluor-Apatit, biphasisches Calciumphosphat, Tetracalciumphosphat (TTCP) und Mischungen davon.

Besonders bevorzugt ist das Calciumphosphat-Material ausgewählt aus der Gruppe bestehend aus Hydroxylapatit, α-Tricalciumphosphat, β-Tricalciumphosphat, biphasisches Calciumphosphat und Mischungen davon.

Unter dem Ausdruck "biphasisches Calciumphosphat" soll im Sinne der vorliegenden Erfindung eine Mischung von β-Tricalciumphosphat und Hydroxylapatit verstanden werden. Eine solche Mischung weist bevorzugt einen Anteil an β-Tricalciumphosphat von 50 Gew.-% und einen Anteil an Hydroxylapatit von 50 Gew.-%, jeweils bezogen auf das Gesamtgewicht der Mischung, auf.

Alternativ oder in Kombination kann die Knochenanwachseinrichtung ein magnesiumhaltiges Material, insbesondere ausgewählt aus der Gruppe bestehend aus Magnesiumhydrogenphosphat (MgHPO₄) in Form der Hydrate oder als wasserfreie Substanz, Trimagnesiumphosphat (Mg₃(PO₄)₂), Magnesiumdihydrogenphosphat (Mg(H₂PO₄)₂) in Form der Hydrate oder als wasserfreie Substanz, Magnesiumchlorid (MgCl₂) in Form der Hydrate oder als wasserfreie Substanz, Magnesiumglycerophosphat, Magnesiumhydroxid (Mg(OH)₂), Magnesiumhydroxidcarbonat (beispielsweise als 4 MgCO₃ × Mg(OH)₂ × 5 H₂O), Magnesiumoxid (MgO), Magnesiumcitrate (Mg₃(C₆H₅O₇)₂ oder Mg(C₆H₆O₇)), Calcium-Magnesiumcarbonat (CaMg(CO₃)₂, Dolomit und Mischungen davon, aufweisen.

Alternativ oder in Kombination kann die Knochenanwachseinrichtung ein Protein aufweisen oder aus einem Protein bestehen. Das Protein kann ausgewählt sein aus der Gruppe bestehend aus Collagen, Gelatine, Elastin, Retikulin, Fibronektin, Laminin, Fibrin, Fibrinogen, Albumin wie Serumalbumin, Salze davon, Stereoisomere, insbesondere Diastereomere, davon und Mischungen davon.

Alternativ oder in Kombination kann die Knochenanwachseinrichtung ein Polysaccharid aufweisen oder aus einem Polysaccharid bestehen. Das Polysaccharid kann ausgewählt sein aus der Gruppe bestehend aus Stärke, Amylose, Amylopektin, Dextran, Dextrin, Cellulose, Methylcellulose, Ethylcellulose, Hydroxymethylcellulose, Hydroxyethylcellulose, Propylcellulose, Hydroxypropylcellulose, Butylcellulose, Carboxymethylcellulose, Carboxyethylcellulose, Algininsäure, Alginate, Chitin, Chitosan, Hyaluronsäure, Dextransulfat, Heparin, Heparansulfat, Chondroitininsulfat wie Chondroitin-4-Sulfat und/oder Chondroitin-6-Sulfat, Dermatansulfat, Keratansulfat, Salze davon, Stereoisomere, insbesondere Diastereomere, davon und Mischungen davon.

Alternativ oder in Kombination kann die Knochenanwachseinrichtung ein synthetisches Polymer aufweisen oder aus einem solchen Polymer bestehen. Das synthetische Polymer kann ausgewählt sein aus der Gruppe bestehend aus Polyetheretherketon (PEEK), Polyethylen, Polyglycolsäure, Polymilchsäure, Poly-ε-caprolacton, Polytrimethylencarbonat, Poly-para-Dioxanon, Poly-3-Hydroxybutansäure, Poly-4-Hydroxybutansäure, Salze davon, Copolymere davon und Mischungen (Blends) davon.

In weiterer Ausgestaltung der Erfindung weist die Knochenanwachseinrichtung ein Metall auf oder besteht aus einem Metall. Das Metall kann insbesondere ausgewählt sein aus der Gruppe bestehend aus Magnesium, Tantal, Titan, Eisen, Zirconium, Niobium, Gold und Platin.

Bevorzugt weist die Knochenanwachseinrichtung Titan auf oder besteht aus Titan.

Insbesondere kann die Knochenanwachseinrichtung eine Metallstruktur, insbesondere eine trabekulare Metallstruktur, aufweisen oder aus einer Metallstruktur, insbesondere trabekularen Metallstruktur, bestehen.

Unter dem Ausdruck "Metallstruktur" soll im Sinne der vorliegenden Erfindung eine Struktur verstanden werden, welche ein Metall aufweist oder aus einem Metall besteht. Bezüglich hierfür geeigneter Metalle wird auf die weiter oben offenbarten Metalle Bezug genommen.

Unter dem Ausdruck "trabekulare Metallstruktur" soll im Sinne der vorliegenden Erfindung eine geometrische, poröse, dreidimensionale Metallgitterstruktur verstanden werden.

Die Metallstruktur ist vorzugsweise mittels eines Laser-Sinter-Prozesses hergestellt oder herstellbar. Insbesondere kann die Metallstruktur gitterförmig gestaltet sein. Weiterhin kann die Metallstruktur beispielsweise offenporig gestaltet sein und insbesondere Poren mit einem Durchmesser von 1,5 mm bis 2,5 mm, und/oder Streben, insbesondere Gitterstreben, bevorzugt mit einer Länge von 0,8 mm bis 1,0 mm, aufweisen.

Bevorzugt handelt es sich bei der Metallstruktur um eine Titanstruktur, insbesondere um eine Titangitterstruktur. Eine entsprechende Titanstruktur ist beispielsweise unter der Bezeichnung Structan^{®} kommerziell erhältlich.

Besonders bevorzugt ist die Knochenanwachseinrichtung als Titanschicht, insbesondere mit einer Dicke von 0,30 mm bis 0,40 mm, bevorzugt 0,35 mm, und/oder einer Porosität, insbesondere Mikroporosität, von bis zu 40%, und/oder mit Poren mit einem mittleren Porendurchmesser, bestimmt mittels Rasterelektronenmikroskopie und/oder Quecksilber-Porosimetrie, von 50 µm bis 200 µm gestaltet. Ein entsprechendes Implantat kann beispielsweise mittels eines von der Anmelderin unter der Bezeichnung Plasmapore^{®} durchgeführten Verfahrens hergestellt werden. Entsprechend kann reines Titanpulver mittels eines Plasmabeschichtungsprozesses unter Vakuum oder Unterdruck mit einer Dicke von 0,35 mm und einer Mikroporosität von bis zu 40% auf die Pyrocarbonschicht, einen Pyrocarbonschichtträger (wie in der vorliegenden Beschreibung offenbart) oder einen Metall- oder Legierungsformkörper (wie in der vorliegenden Beschreibung offenbart) aufgebracht werden, wobei die so erzeugte Titanschicht vorzugsweise Poren mit einem mittleren Durchmesser von 50 µm bis 200 µm aufweist, welche in vorteilhafter Weise eine direkte Knochenapposition bewirken können.

In weiterer Ausgestaltung der Erfindung weist die Knochenanwachseinrichtung eine Legierung auf oder besteht aus einer Legierung. Die Legierung kann insbesondere ausgewählt sein aus der Gruppe bestehend aus Magnesiumlegierung, Tantallegierung, Titanlegierung, Chrom-Cobalt-Legierung, Chrom-Cobalt-Molybdän-Legierung, Eisenlegierung, Stahl wie Edelstahl, Zirkoniumlegierung, Niobiumlegierung, Goldlegierung, Platinlegierung und Mischungen davon.

Unter dem Ausdruck "Edelstahl" soll im Sinne der vorliegenden Erfindung (in Übereinstimmung nach EN 10020) ein legierter oder unlegierter Stahl mit besonderem Reinheitsgrad, beispielsweise mit einem Schwefel- und/oder Phosphormassenanteil ≤ 0,025 %, insbesondere < 0,025 %, verstanden werden.

Beispielsweise kann es sich bei dem Stahl um einen Stahl mit der Werkstoffkurzbezeichnung X12Cr13 (Werkstoffnummer 1.4006) handeln. Hierbei handelt es sich um einen martensitischen Stahl mit einem Kohlenstoffmassenanteil von 0,08 % bis 0,15 %, einem Chrommassenanteil von 11,5 % bis 13,5 % sowie einem Nickelmasseanteil von ≤ 0,75 %.

Alternativ kann es sich bei dem Stahl um einen martensitischen korrosionsbeständigen Stahl mit der Werkstoffkurzbezeichnung X12CrS13 (Werkstoffnummer 1.4005) handeln. Dieser Stahl weist einen Kohlenstoffmassenanteil von 0,08 % bis 0,15 %, einen Chrommassenanteil von 12,0 % bis 14,0 % sowie eine Molybdänmassenanteil ≤ 0,60 % sowie optional einen Schwefelmassenanteil von 0,15 % bis 0,35 % auf.

Insbesondere kann die Knochen- und/oder Knorpelanwachseinrichtung eine Legierungsstruktur, insbesondere eine trabekulare Legierungsstruktur, aufweisen oder aus einer Legierungsstruktur, insbesondere trabekularen Legierungsstruktur, bestehen.

Unter dem Ausdruck "Legierungsstruktur" soll im Sinne der vorliegenden Erfindung eine Struktur verstanden werden, welche eine Legierung aufweist oder aus einer Legierung besteht. Bezüglich hierfür geeigneter Legierungen wird auf die weiter oben offenbarten Legierungen Bezug genommen.

Unter dem Ausdruck "trabekulare Legierungsstruktur" soll im Sinne der vorliegenden Erfindung eine geometrische, poröse, dreidimensionale Gitterstruktur aus einer Legierung verstanden werden.

Die Legierungsstruktur kann insbesondere mittels eines Laser-Sinter-Prozesses hergestellt oder herstellbar sein. Insbesondere kann die Legierungsstruktur gitterförmig gestaltet sein. Weiterhin kann die Legierungsstruktur beispielsweise offenporig gestaltet sein und insbesondere Poren mit einem Durchmesser, insbesondere mittleren Durchmesser, bestimmt mittels Rasterelektronenmikroskopie und/oder Quecksilber-Porosimetrie, von 1,5 mm bis 2,5 mm, und/oder Streben, insbesondere Gitterstreben, bevorzugt mit einer Länge von 0,8 mm bis 1,0 mm, aufweisen.

Weiterhin kann das Implantat, insbesondere die Pyrocarbonschicht und/oder die Knochenanwachseinrichtung, wenigstens einen Wirkstoff aufweisen. Alternativ oder in Kombination kann ein Pyrocarbonschichträger (wie in der vorliegenden Beschreibung offenbart) und/oder ein Metall- und/oder Legierungsformkörper (wie in der vorliegenden Beschreibung offenbart) wenigstens einen Wirkstoff aufweisen. Der Wirkstoff kann ausgewählt sein aus der Gruppe bestehend aus antimikrobieller, insbesondere antibiotischer, Wirkstoff, wundheilungsfördernder Wirkstoff, desinfizierender Wirkstoff, entzündungshemmender bzw. anti-inflammatorischer Wirkstoff, blutgerinnungsfördernder Wirkstoff, Wachstumsfaktor wie Knochenwachstumsfaktor (osteoinduktiver Faktor) und/oder Knorpelwachstumsfaktor (chondrogener Faktor), zelldifferenzierender Faktor, zelladhäsiver Faktor, zellrekrutierender Faktor, Zellrezeptor, zellbindender Faktor, Zytokin, Peptid, Strukturprotein, extrazelluläres Protein wie Collagen, Elastin, Retikulin und dergleichen, Serumprotein wie Albumin, Polysaccharid wie Hyaluronsäure, Oligonukleotid, Polynukleotid, DNA, RNA, Salze davon, Stereoisomere, insbesondere Diastereomere, davon und Mischungen davon.

Weiterhin kann das Implantat Vertiefungen und/oder Durchbrüche, insbesondere in Form von Bohrungen, aufweisen. Die Durchbrüche schaffen mit besonderem Vorteil zusätzliche Fixierungsmöglichkeiten des Implantats, beispielsweise mittels Pins und/oder Schrauben.

Bei dem Implantat gemäß der vorliegenden Erfindung kann es sich insbesondere um ein Knochentotalersatzimplantat, d.h. um ein Implantat, welches zum totalen oder vollständigen Ersatz eines Knochens und/oder Knorpels ausgebildet ist, oder um ein Knochenteilersatzimplantat, d.h. um ein Implantat, welches lediglich zum teilweisen Ersatz eines Knochen und/oder Knorpels ausgebildet ist, handeln. Beispielsweise kann es sich bei dem Implantat um ein Knochentotalersatzimplantat eines Wibelkörpers handeln.

Vorzugsweise handelt es sich bei dem Implantat um ein Gelenkimplantat, insbesondere Hüftgelenk- oder Kniegelenkimplantat. Dabei kann es sich bei dem Implantat um ein Gelenktotalersatzimplantat, insbesondere Hüftgelenktotal- oder Kniegelenktotalersatzimplantat, d.h. um ein Implantat, welches zum totalen oder vollständigen Ersatz eines Gelenks, insbesondere Hüftgelenks oder Kniegelenks, ausgebildet ist, oder um ein Gelenkteilersatzimplantat, insbesondere Hüftgelenkteil- oder Kniegelenkteilersatzimplantat, d.h. um ein Implantat, welches lediglich zum Teilersatz eines Gelenks, insbesondere Hüftgelenks oder Kniegelenks, ausgebildet ist, handeln.

Das Implantat kann insbesondere als künstliche Gelenkpfanne, künstliches Gelenkpfannen-Inlay, Abstützschale oder als Teil eines solchen Implantats gestaltet sein.

Weiterhin kann es sich bei dem Implantat um ein Implantat zum Ersatz, insbesondere vollständigen Ersatz oder Teilersatz, einer Kondyle oder eines Tibiaplateaus handeln.

Weiterhin kann es sich bei dem Implantat um ein Implantat zum Ersatz, insbesondere vollständigen Ersatz oder Teilersatz, eines Patella-Gleitlagers oder Trochlea-Gleitlagers handeln.

Weiterhin kann es sich bei dem Implantat um ein Implantat zum Ersatz, insbesondere vollständigen Ersatz oder Teilersatz, eines Sprunggelenks handeln.

Weiterhin kann es sich bei dem Implantat um ein Implantat zum Ersatz, insbesondere vollständigen Ersatz oder Teilersatz, eines Meniskus handeln.

Weiterhin kann es sich bei dem Implantat um ein Implantat zum Behandeln oder Versorgen von osteochondralen, insbesondere begrenzten osteochondralen, Läsionen handeln.

Weiterhin kann es sich bei dem Implantat um ein Wirbelsäulenimplantat, insbesondere um ein Wirbelkörper- oder Bandscheibenimplantat, handeln. Bevorzugt handelt es sich bei dem Implantat um ein Implantat zum Ersatz, insbesondere vollständigen Ersatz oder Teilersatz, eines Wirbelkörpers oder einer Bandscheibe.

Weitere Merkmale und Vorteile der Erfindung ergeben sich aus den Ansprüchen sowie aus der nachfolgenden Beschreibung von bevorzugten Ausführungsformen der Erfindung, welche anhand eines Ausführungsbeispiels sowie von Figuren und zugehörigen Figurenbeschreibungen näher erläutert werden. Dabei können Merkmale der Erfindung jeweils für sich alleine oder in Kombination miteinander verwirklicht sein. Die nachfolgend beschriebenen Ausführungsformen dienen der weiteren Erläuterung der Erfindung, ohne diese hierauf zu beschränken.

### KURZBESCHREIBUNG DER ZEICHNUNGEN

In den Figuren ist schematisch Folgendes gezeigt:
- Fig. 1a:: ein Längsschnitt einer Ausführungsform eines nicht erfindungsgemäßen Implantats,
- Fig. 1b:: ein Längsschnitt einer weiteren Ausführungsform eines nicht erfindungsgemäßen Implantats,
- Fig. 1c:: ein Längsschnitt einer weiteren Ausführungsform eines nicht erfindungsgemäßen Implantats,
- Fig. 2a:: ein Längsschnitt einer weiteren Ausführungsform eines erfindungsgemäßen Implantats,
- Fig. 2b:: ein Längsschnitt einer weiteren Ausführungsform eines erfindungsgemäßen Implantats,
- Fig. 2c:: ein Längsschnitt einer weiteren Ausführungsform eines nicht erfindungsgemäßen Implantats und
- Fig. 3:: eine weitere Ausführungsform eines erfindungsgemäßen Implantats. DETAILLIERTE BESCHREIBUNG DER AUSFÜHRUNGSBEISPIELE

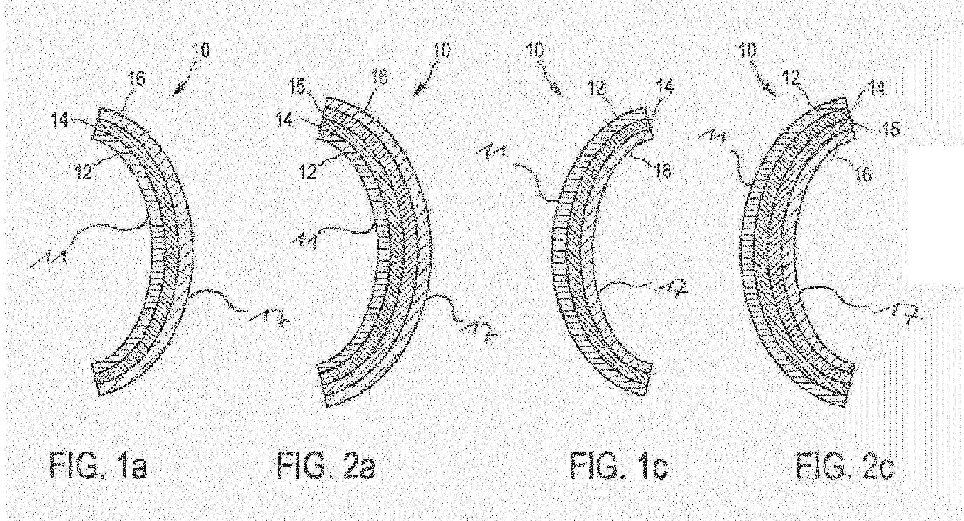

Fig. 1a zeigt schematisch einen Längsschnitt einer Ausführungsform eines nicht erfindungsgemäßen Implantats 10.

Das gezeigte Implantat 10 weist eine Pyrocarbonschicht 12, einen Pyrocarbonschichtträger 14 sowie eine Knochenanwachseinrichtung 16 auf. Der Pyrocarbonschichtträger 14 ist zwischen der Pyrocarbonschicht 12 und der Knochenanwachseinrichtung 16 angeordnet. Der Pyrocarbonschichtträger 14 steht dabei bevorzugt sowohl mit der Pyrocarbonschicht 12 als auch mit der Knochenanwachseinrichtung 16 in unmittelbarem Kontakt. Die Pyrocarbonschicht 12 bildet eine konkave Außenfläche 11 des Implantats 10, während die Knochenanwachseinrichtung 16 eine konvexe Außenfläche 17 des Implantats 10 bildet.

Die Pyrocarbonschicht 12 ist vorzugsweise zum Artikulieren mit einem Knochen und/oder Knorpel ausgebildet. Hierzu besitzt die Pyrocarbonschicht 12 zweckmäßigerweise eine glatte und insbesondere frei zugängliche Außenoberfläche. Pyrocarbon ist insbesondere aufgrund seiner tribologischen und mechanischen, insbesondere flexiblen oder elastischen, Eigenschaften in besonderer Weise zum Artikulieren mit Knochen und/oder Knorpel geeignet.

Die Pyrocarbonschicht 12 kann eine Schichtdicke von 0,5 mm bis 1 mm aufweisen.

Bei dem Pyrocarbonschichtträger 14 handelt es sich vorzugsweise um einen Graphitformkörper, d.h. um einen Formkörper, welcher Graphit aufweist oder aus Graphit besteht. Der Pyrocarbonschichtträger 14 kann eine Dicke von 0,5 mm bis 1,5 mm aufweisen.

Die Knochenanwachseinrichtung 16 ist vorzugsweise schichtförmig, insbesondere in Form einer Beschichtung, ausgebildet.

Die Knochenanwachseinrichtung 16 kann ein Calciumphosphat-Material, wie beispielsweise Hydroxylapatit, α-Tricalciumphosphat, β-Tricalciumphosphat oder biphasisches Calciumphosphat, aufweisen oder aus einem solchen Material bestehen. Vorzugsweise besitzt die Knochenanwachseinrichtung 16 in diesem Fall eine Schichtdicke von 5 µm bis 50 µm, bevorzugt 5 µm bis 30 µm. Eine solche Schichtdicke hat insbesondere den Vorteil, dass hierdurch die tribologischen und/oder mechanischen Eigenschaften der Pyrocarbonschicht 12 nicht beeinträchtigt werden und trotzdem eine ausreichende Implantatstabilität erzielbar ist.

Alternativ kann es bevorzugt sein, wenn die Knochenanwachseinrichtung 16 ein Metall oder eine Legierung aufweist oder aus einem Metall oder einer Legierung besteht. In diesem Fall kann es vorteilhaft sein, wenn die Knochenanwachseinrichtung 16 eine Schichtdicke von 250 µm bis 700 µm, insbesondere 300 µm bis 400 µm, aufweist. Dadurch kann eine Beeinträchtigung der tribologischen und/oder mechanischen Eigenschaften der Pyrocarbonschicht 12 vermieden und trotzdem eine ausreichende Implantatstabilität erzielt werden.

Liegt die Knochenanwachseinrichtung 16 dagegen als Metall- oder Legierungsformkörper vor, kann eine Dicke von 0,2 mm bis 2 mm vorteilhaft sein, um eine Beeinträchtigung der tribologischen und/oder mechanischen Eigenschaften der Pyrocarbonschicht 12 zu vermeiden und trotzdem eine ausreichende Implantatstabilität zu erzielen.

Die Knochenanwachseinrichtung 16 weist bevorzugt eine strukturierte, insbesondere aufgeraute, und/oder eine offenporig gestaltete Oberfläche, insbesondere Außenoberfläche, auf. Dadurch kann mit besonderem Vorteil ein Anwachsen von Knochen- und/oder Knorpelgewebe an die Knochenanwachseinrichtung 16 und/oder ein Einwachsen von Knochen- und/oder Knorpelgewebe in die Knochenanwachseinrichtung 16 hinein erzielt werden. Dies wiederrum ermöglicht eine sichere Befestigung des Implantats 10 im Körper eines Patienten.

Das Implantat 10, insbesondere die Pyrocarbonschicht 12, der Pyrocarbonschichtträger 14 sowie die Knochenanwachseinrichtung 16, sind vorzugsweise schalenförmig oder kugelschalensegmentförmig, insbesondere halbkugelschalenförmig, gestaltet. Eine derartige Ausgestaltung des Implantats 10, insbesondere der Pyrocarbonschicht 12, des Pyrocarbonschichtträgers 14 sowie der Knochenanwachseinrichtung 16, ist insbesondere bei der Behandlung von Knochengelenksdefekten von Vorteil.

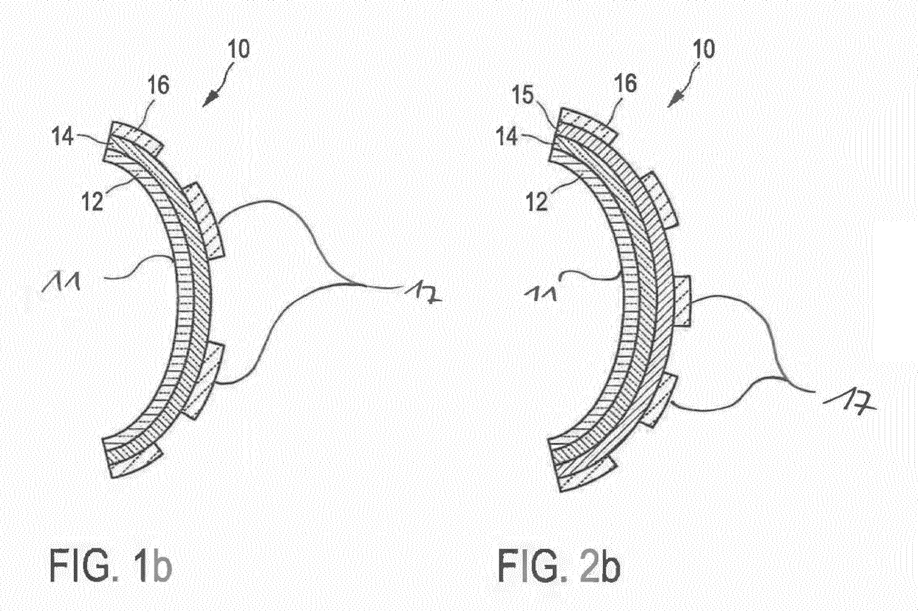

Fig. 1b zeigt schematisch einen Längsschnitt einer weiteren Ausführungsform eines nicht erfindungsgemäßen Implantats 10.

Das gezeigte Implantat 10 weist eine Pyrocarbonschicht 12, einen Pyrocarbonschichtträger 14 sowie eine Knochenanwachseinrichtung 16 auf.

Im Gegensatz zu der in Fig. 1a dargestellten Ausführungsform eines nicht erfindungsgemäßen Implantats ist die Knochenanwachseinrichtung 16 bei dem Implantat 10 gemäß Fig. 1b lediglich abschnitts- oder bereichsweise auf dem Pyrocarbonschichtträger 14 ausgebildet. Somit bildet die Knochenanwachseinrichtung 16 nur teilweise, d.h. nur abschnitts- oder bereichsweise, eine konvexe Außenfläche 17 des Implantats 10. Dadurch bleiben Elastizität und Flexibilität der Schichten 12 und 14 erhalten, was in der Gleitpaarung mit dem natürlichen Knorpel- oder Knochengewebe besonders vorteilhaft ist.

Bezüglich weiterer Merkmale und Vorteile des in Fig. 1b gezeigten Implantats 10, insbesondere der Pyrocarbonschicht 12, des Pyrocarbonschichtträgers 14 sowie der Knochenanwachseinrichtung 16, wird zur Vermeidung von Wiederholungen vollständig auf die Figurenbeschreibung der Fig. 1a Bezug genommen. Die dort in Bezug auf das Implantat 10, insbesondere die Pyrocarbonschicht 12, den Pyrocarbonschichtträger 14 sowie die Knochenanwachseinrichtung 16, beschriebenen Merkmale und Vorteile gelten sinngemäß auch für das Implantat 10, insbesondere die Pyrocarbonschicht 12, den Pyrocarbonschichtträger 14 sowie die Knochenanwachseinrichtung 16, gemäß Fig. 1b.

Fig. 1c zeigt schematisch einen Längsschnitt einer weiteren Ausführungsform eines nicht erfindungsgemäßen Implantats 10.

Das gezeigte Implantat 10 weist eine Pyrocarbonschicht 12, einen Pyrocarbonschichtträger 14 sowie eine Knochenanwachseinrichtung 16 auf. Im Gegensatz zu dem in Fig. 1a gezeigten Implantat bildet die Pyrocarbonschicht 12 bei dem in Fig. 1c gezeigten Implantat eine konvexe Außenfläche 11, während die Knochenanwachseinrichtung 16 eine konkave Außenfläche 17 bildet.

Bezüglich weiterer Merkmale und Vorteile des in Fig. 1c gezeigten Implantats 10, insbesondere der Pyrocarbonschicht 12, des Pyrocarbonschichtträgers 14 sowie der Knochenanwachseinreichung 16, wird zur Vermeidung von Wiederholungen ebenfalls vollständig auf die Figurenbeschreibung der Fig. 1a Bezug genommen. Die dort in Bezug auf das Implantat 10, insbesondere die Pyrocarbonschicht 12, den Pyrocarbonschichtträger 14 sowie die Knochenanwachseinreichung 16, beschriebenen Merkmale und Vorteile gelten sinngemäß auch für das Implantat 10, insbesondere die Pyrocarbonschicht 12, den Pyrocarbonschichtträger 14 sowie die Knochenanwachseinrichtung 16, gemäß Fig. 1c.

Die in den Fig. 1a bis 1c gezeigten Implantate sind vorzugsweise frei von einem Metall- oder Legierungsformkörper.

Fig. 2a zeigt schematisch einen Längsschnitt einer weiteren Ausführungsform eines erfindungsgemäßen Implantats 10.

Das gezeigte Implantat 10 weist eine Pyrocarbonschicht 12, einen Pyrocarbonschichtträger 14, einen Metall- oder Legierungsformkörper 15 sowie eine Knochenanwachseinrichtung 16 auf. Die Pyrocarbonschicht 12 bildet eine konkave Außenfläche 11 des Implantats 10, während die Knochenanwachseinrichtung 16 eine konvexe Außenfläche 17 des Implantats 10 bildet.

Der Pyrocarbonschichtträger 14 ist zwischen der Pyrocarbonschicht 12 und dem Metall- oder Legierungsformkörper 15 angeordnet. Dabei steht der Pyrocarbonschichtträger sowohl mit der Pyrocarbonschicht 12 als auch mit dem Metall- oder Legierungsformkörper 15 in direktem Kontakt.

Der Metall- oder Legierungsformkörper 15 ist zwischen dem Pyrocarbonschichtträger 14 und der Knochenanwachseinrichtung 16 angeordnet. Bevorzugt steht der Metall- oder Legierungsformkörper 15 dabei sowohl mit dem Pyrocarbonschichtträger 14 als auch mit der Knochenanwachseinrichtung 16 in direktem Kontakt.

Bezüglich weiterer Merkmale und Vorteile des Implantats 10, insbesondere der Pyrocarbonschicht 12, des Pyrocarbonschichtträgers 14 sowie der Knochenanwachseinrichtung 16, wird zur Vermeidung von Wiederholungen vollständig auf die Figurenbeschreibung der Fig. 1a Bezug genommen. Die dort in Bezug auf das Implantat 10, insbesondere die Pyrocarbonschicht 12, den Pyrocarbonschichtträger 14 sowie die Knochenanwachseinrichtung 16, beschriebenen Merkmale und Vorteile gelten sinngemäß auch für das Implantat 10, insbesondere die Pyrocarbonschicht 12, den Pyrocarbonschichtträger 14 sowie die Knochenanwachseinrichtung 16, gemäß Fig. 2a.

Fig. 2b zeigt schematisch einen Längsschnitt einer weiteren Ausführungsform eines erfindungsgemäßen Implantats 10.

Das gezeigte Implantat 10 weist eine Pyrocarbonschicht 12, einen Pyrocarbonschichtträger 14, einen Metall- oder Legierungsformkörper 15 sowie eine Knochenanwachseinrichtung 16 auf.

Im Gegensatz zu der in Fig. 2a dargestellten Ausführungsform eines erfindungsgemäßen Implantats ist die Knochenanwachseinrichtung 16 bei dem Implantat 10 gemäß Fig. 2b lediglich abschnitts- oder bereichsweise auf dem Metall- oder Legierungsformkörper 15 ausgebildet. Somit bildet die Knochenanwachseinrichtung 16 nur teilweise, d.h. nur abschnitts- oder bereichsweise, eine konvexe Außenfläche 17 des Implantats 10. Dadurch bleiben die Elastizität und Flexibilität insbesondere der Schichten 12 und 14 erhalten, was in der Gleitpaarung mit dem natürlichen Knorpel- oder Knochengewebe besonders vorteilhaft ist.

Bezüglich weiterer Merkmale und Vorteile des in Fig. 2b gezeigten Implantats 10, insbesondere der Pyrocarbonschicht 12, des Pyrocarbonschichtträgers 14, des Metall- oder Legierungsformkörpers 15 sowie der Knochenanwachseinrichtung 16, wird zur Vermeidung von Wiederholungen vollständig auf die Figurenbeschreibung der Fig. 1a und 2a Bezug genommen. Die dort in Bezug auf das Implantat 10, insbesondere die Pyrocarbonschicht 12, den Pyrocarbonschichtträger 14, den Metall- oder Legierungsformkörper 15 sowie die Knochenanwachseinrichtung 16, beschriebenen Merkmale und Vorteile gelten sinngemäß auch für das Implantat 10, insbesondere die Pyrocarbonschicht 12, den Pyrocarbonschichtträger 14, den Metall- oder Legierungsformkörper 15 sowie die Knochenanwachseinrichtung 16, gemäß Fig. 2b.

Fig. 2c zeigt schematisch einen Längsschnitt einer weiteren Ausführungsform eines nicht erfindungsgemäßen Implantats 10.

Das gezeigte Implantat 10 weist eine Pyrocarbonschicht 12, einen Pyrocarbonschichtträger 14, einen Metall- oder Legierungsformkörper 15 sowie eine Knochenanwachseinrichtung 16 auf. Im Gegensatz zu dem in Fig. 2a gezeigten Implantat, bildet die Pyrocarbonschicht 12 bei dem in Fig. 2c gezeigten Implantat 10 eine konvexe Außenfläche 11, während die Knochenanwachseinrichtung 16 ein konkave Außenfläche 17 bildet.

Bezüglich weiterer Merkmale und Vorteile des in Fig. 2c gezeigten Implantats 10, insbesondere der Pyrocarbonschicht 12, des Pyrocarbonschichtträgers 14, des Metall- oder Legierungsformskörpers 15 sowie der Knochenanwachseinrichtung 16, wird zur Vermeidung von Wiederholungen ebenfalls vollständig auf die Figurenbeschreibung der Fig. 1a und 2a Bezug genommen. Die dort in Bezug auf das Implantat, insbesondere die Pyrocarbonschicht 12, den Pyrocarbonschichtträger 14, den Metall- oder Legierungsformskörper 15 sowie die Knochenanwachseinrichtung 16, beschriebenen Merkmale und Vorteile gelten sinngemäß auch für das Implantat 10, insbesondere die Pyrocarbonschicht 12, den Pyrocarbonschichtträger 14, den Metall- oder Legierungsformskörper 15 sowie die Knochenanwachseinrichtung 16, gemäß Fig. 2c.

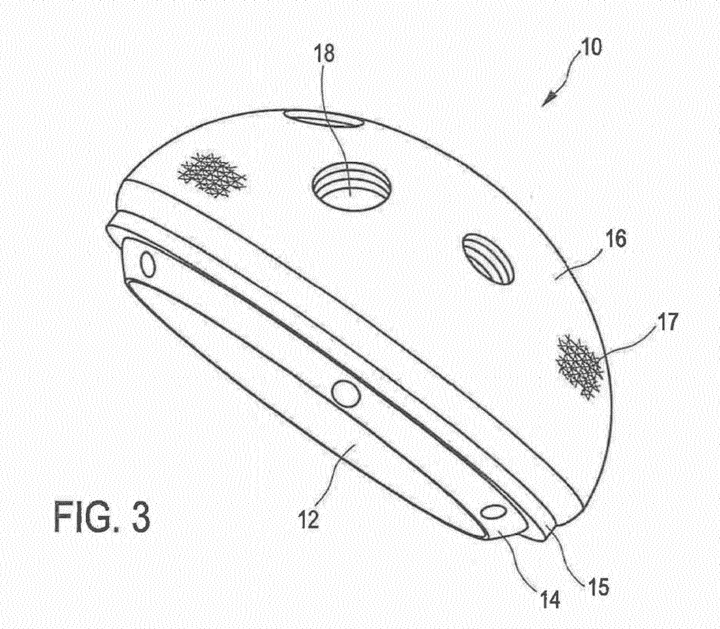

Fig. 3 zeigt schematisch eine weitere Ausführungsform eines erfindungsgemäßen Implantats 10.

Das gezeigte Implantat 10 weist eine Pyrocarbonschicht 12, einen Pyrocarbonschichtträger 14, einen Metall- oder Legierungsformkörper 15 sowie eine Knochenanwachseinrichtung 16 auf.

Die Knochenanwachseinrichtung 16 besitzt eine strukturierte und/oder offenporig gestaltete Fläche oder Oberfläche, insbesondere Außenfläche oder Außenoberfläche, 17.

Die Fläche bzw. Oberfläche 17 bewirkt mit besonderem Vorteil das An- und/oder Hineinwachsen von Knochen- und/oder Knorpelgewebe an und/oder in die Knochenanwachseinrichtung 16 hinein. Dadurch ist eine sichere Aufhängung des Implantats 10 im Körper eines Patienten erzielbar.

Je nach gewähltem Material kann die Knochenanwachseinrichtung 16 eine Dicke, insbesondere Schichtdicke, von 5 µm bis 50 µm oder 250 µm bis 700 µm aufweisen.

Des Weiteren kann das Implantat 10 Durchbrüche 18, insbesondere in Form von Bohrungen, aufweisen. Die Durchbrüche 18 eröffnen mit besonderem Vorteil zusätzliche Fixierungsmöglichkeiten des Implantats, beispielsweise mittels Pins oder Schrauben. Alternativ oder im Kombination kann das Implantat 10 Vertiefungen aufweisen (nicht dargestellt).

Bezüglich weiterer Merkmale und Vorteile des Implantats 10, insbesondere der Pyrocarbonschicht 12, des Pyrocarbonschichtträgers 14, des Metall- oder Legierungsformkörpers 15 sowie der Knochenanwachseinrichtung 16, wird zur Vermeidung von Wiederholungen vollständig auf die bisherigen Figurenbeschreibungen Bezug genommen. Die dort in Bezug auf das Implantat 10, insbesondere die Pyrocarbonschicht 12, den Pyrocarbonschichtträger 14, den Metall- oder Legierungsformkörper 15 sowie die Knochenanwachseinrichtung 16, beschriebenen Merkmale und Vorteile gelten sinngemäß auch für das Implantat gemäß Fig. 3.

## Patentansprüche

1. Implantat, aufweisend eine Pyrocarbonschicht und eine Knochenanwachseinrichtung, wobei die Pyrocarbonschicht eine konkave Außenfläche oder konkave Außenschicht und die Knochenanwachseinrichtung eine konvexe Außenfläche oder konvexe Außenschicht des Implantats bildet, **dadurch gekennzeichnet, dass** das Implantat ferner einen Metall- oder Legierungsformkörper aufweist, wobei der Metall- oder Legierungsformkörper zwischen der Pyrocarbonschicht und der Knochenanwachseinrichtung angeordnet ist.

2. Implantat nach Anspruch 1, **dadurch gekennzeichnet, dass** die Pyrocarbonschicht auf einem Pyrocarbonschichtträger, insbesondere schalenförmigen oder kugelschalensegmentförmigen Pyrocarbonschichtträger, ausgebildet ist.

3. Implantat nach Anspruch 2, **dadurch gekennzeichnet, dass** es sich bei dem Pyrocarbonschichtträger um einen Graphitformkörper handelt.

4. Implantat nach Anspruch 2 oder 3, **dadurch gekennzeichnet, dass** die Knochenanwachseinrichtung wenigstens teilweise auf dem Pyrocarbonschichtträger ausgebildet ist.

5. Implantat nach einem der vorhergehenden Ansprüche, **dadurch gekennzeichnet, dass** die Knochenanwachseinrichtung eine strukturierte Oberfläche aufweist und/oder offenporig gestaltet ist, insbesondere Poren mit einem mittleren Porendurchmesser von 50 µm bis 1500 µm, bevorzugt 100 µm bis 1000 µm, besonders bevorzugt 100 µm bis 550 µm, aufweist.

6. Implantat nach einem der vorhergehenden Ansprüche, **dadurch gekennzeichnet, dass** die Knochenanwachseinrichtung schichtförmig, insbesondere in Form einer Beschichtung, gestaltet ist.

7. Implantat nach einem der vorhergehenden Ansprüche, **dadurch gekennzeichnet, dass** die Knochenanwachseinrichtung eine Schichtdicke von 5 µm bis 50 µm, insbesondere 5 µm bis 40 µm, bevorzugt 5 µm bis 30 µm, aufweist.

8. Implantat nach einem der Ansprüche 1 bis 6, **dadurch gekennzeichnet, dass** die Knochenanwachseinrichtung eine Schichtdicke von 250 µm bis 700 µm, insbesondere 300 µm bis 400 µm, bevorzugt 350 µm, aufweist.

9. Implantat nach einem der vorhergehenden Ansprüche, **dadurch gekennzeichnet, dass** der Metall- oder Legierungsformkörper schalenförmig oder kugelschalensegmentförmig gestaltet ist.

10. Implantat nach einem der vorhergehenden Ansprüche, **dadurch gekennzeichnet, dass** die Knochenanwachseinrichtung wenigstens teilweise auf dem Metall- oder Legierungsformkörper ausgebildet ist.

11. Implantat nach einem der Ansprüche 1 bis 5, **dadurch gekennzeichnet, dass** es sich bei der Knochenanwachseinrichtung um einen Metall- oder Legierungsformkörper, insbesondere schalenförmigen oder kugelschalensegmentförmigen Metall- oder Legierungsformkörper, handelt.

12. Implantat nach Anspruch 11, **dadurch gekennzeichnet, dass** der Metall- oder Legierungsformkörper eine Dicke von 0,2 mm bis 2 mm, insbesondere 0,5 mm aufweist.

13. Implantat nach einem der vorhergehenden Ansprüche, **dadurch gekennzeichnet, dass** die Knochenanwachseinrichtung ein calciumhaltiges Material, bevorzugt ein Calciumphosphat-Material, insbesondere ausgewählt aus der Gruppe bestehend aus Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), α-Tricalciumphosphat (α-TCP), β-Tricalciumphosphat (β-TCP), amorphes Calciumphosphat (ACP), Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Fluorapatit, Chlorapatit, Carbonat-Fluor-Apatit, biphasisches Calciumphosphat, Tetracalciumphosphat (TTCP), Calciumsulfat (CaSO₄), Calciumsulfat-Hemihydrat (CaSO₄ × 0.5 H₂O), Calciumsulfat-Dihydrat (CaSO₄ × 2 H₂O), Calciumoxid (CaO), Calciumhydroxid (Ca(OH)₂), Calciumcarbonat (CaCO₃), Calciumglycerophosphat, Calciumcitrat, Calciumlactat, Calciumacetat, Calciumtartrat, Calciumchlorid (CaCl₂), Calciumsilicate und Mischungen davon, aufweist.

14. Implantat nach einem der Ansprüche 1 bis 12, **dadurch gekennzeichnet, dass** die Knochenanwachseinrichtung ein Metall oder eine Legierung, insbesondere ausgewählt aus der Gruppe bestehend aus Magnesium, Tantal, Titan, Nickel, Magnesiumlegierung, Tantallegierung, Titanlegierung, Chrom-Cobalt-Legierung, Chrom-Cobalt-Molybdän-Legierung, Stahl wie Edelstahl und Kombinationen davon, aufweist.

## Claims

1. Implant comprising a pyrocarbon layer and a bone take means, wherein the pyrocarbon layer forms a concave exterior surface or concave exterior layer of the implant and the bone take means forms a convex exterior surface or convex exterior layer of the implant, characterized that the implant further comprises a metal or alloy shaped body, wherein the metal or alloy shaped body is arranged between the pyrocarbon layer and the bone take means.

2. Implant according to Claim 1, **characterized in that** the pyrocarbon layer is formed on a pyrocarbon layer support, in particular a pyrocarbon layer support in the form of a shell or in the form of a spherical shell segment.

3. Implant according to Claim 2, **characterized in that** the pyrocarbon layer support is a graphite shaped body.

4. Implant according to Claim 2 or 3, **characterized in that** the bone take means is at least partially formed on the pyrocarbon layer support.

5. Implant according to any of the preceding claims, **characterized in that** the bone take means has a structured surface and/or is open-pored, in particular has pores having a mean pore diameter of from 50 µm to 1500 µm, preferably 100 µm to 1000 µm, particularly preferably 100 µm to 550 µm.

6. Implant according to any of the preceding claims, **characterized in that** the bone take means is in layer form, in particular in the form of a coating.

7. Implant according to any of the preceding claims, **characterized in that** the bone take means has a layer thickness of from 5 µm to 50 µm, in particular 5 µm to 40 µm, preferably 5 µm to 30 µm.

8. Implant according to any of Claims 1 to 6, **characterized in that** the bone take means has a layer thickness of from 250 µm to 700 µm, in particular 300 µm to 400 µm, preferably 350 µm.

9. Implant according to any of the preceding claims, **characterized in that** the metal or alloy shaped body is in the form of a shell or in the form of a spherical shell segment.

10. Implant according to any of the preceding claims, **characterized in that** the bone take means is at least partially formed on the metal or alloy shaped body.

11. Implant according to any of Claims 1 to 5, **characterized in that** the bone take means is a metal or alloy shaped body, in particular a metal or alloy shaped body in the form of a shell or in the form of a spherical shell segment.

12. Implant according to Claim 11, **characterized in that** the metal or alloy shaped body has a thickness of from 0.2 mm to 2 mm, in particular 0.5 mm.

13. Implant according to any of the preceding claims, **characterized in that** the bone take means comprises a calcium-containing material, preferably a calcium phosphate material, in particular selected from the group consisting of monocalcium phosphate monohydrate (MCPM), monocalcium phosphate anhydride (MCPA), dicalcium phosphate anhydride (DCPA), dicalcium phosphate dihydrate (DCPD), octacalcium phosphate (OCP), α-tricalcium phosphate (α-TCP), β-tricalcium phosphate (β-TCP), amorphous calcium phosphate (ACP), hydroxyapatite (HA), calcium-deficient hydroxyapatite (CdHA), substituted hydroxyapatite, non-stoichiometric hydroxyapatite, nanoscale hydroxyapatite, fluorapatite, chlorapatite, carbonate-fluorapatite, biphasic calcium phosphate, tetracalcium phosphate (TTCP), calcium sulfate (CaSO₄), calcium sulfate hemihydrate (CaSO₄ × 0.5 H₂O), calcium sulfate dihydrate (CaSO₄ × 2 H₂O), calcium oxide (CaO), calcium hydroxide (Ca(OH)₂), calcium carbonate (CaCO₃), calcium glycerophosphate, calcium citrate, calcium acetate, calcium tartrate, calcium chloride (CaCl₂), calcium silicates and mixtures thereof.

14. Implant according to any of Claims 1 to 12, **characterized in that** the bone take means comprises a metal or an alloy, in particular selected from the group consisting of magnesium, tantalum, titanium, nickel, magnesium alloy, tantalum alloy, titanium alloy, chromium-cobalt alloy, chromium-cobalt-molybdenum alloy, steel such as stainless steel, and combinations thereof.

## Revendications

1. Implant présentant une couche de pyrocarbone et un dispositif de croissance osseuse, la couche de pyrocarbone formant une surface extérieure concave ou une couche extérieure concave et le dispositif de croissance osseuse formant une surface extérieure convexe ou une couche extérieure convexe de l'implant, **caractérisé en ce que** l'implant présente en outre un corps moulé de métal ou d'alliage, le corps moulé de métal ou d'alliage étant disposé entre la couche de pyrocarbone et le dispositif de croissance osseuse.

2. Implant selon la revendication 1, **caractérisé en ce que** la couche de pyrocarbone est réalisée sur un support de couche de pyrocarbone, en particulier un support de couche de pyrocarbone en forme de coquille ou en forme de segment de coquille sphérique.

3. Implant selon la revendication 2, **caractérisé en ce que** le support de couche de pyrocarbone est un corps moulé de graphite.

4. Implant selon la revendication 2 ou 3, **caractérisé en ce que** le dispositif de croissance osseuse est réalisé au moins partiellement sur le support de couche de pyrocarbone.

5. Implant selon l'une quelconque des revendications précédentes, **caractérisé en ce que** le dispositif de croissance osseuse présente une surface structurée et/ou est réalisé à pores ouverts, en particulier présente des pores dotés d'un diamètre moyen de pores de 50 µm à 1 500 µm, préférablement de 100 µm à 1 000 µm, particulièrement préférablement de 100 µm à 550 µm.

6. Implant selon l'une quelconque des revendications précédentes, **caractérisé en ce que** le dispositif de croissance osseuse est réalisé sous forme de couches, en particulier sous la forme d'un revêtement.

7. Implant selon l'une quelconque des revendications précédentes, **caractérisé en ce que** le dispositif de croissance osseuse présente une épaisseur de couche de 5 µm à 50 µm, en particulier de 5 µm à 40 µm, préférablement de 5 µm à 30 µm.

8. Implant selon l'une quelconque des revendications 1 à 6, **caractérisé en ce que** le dispositif de croissance osseuse présente une épaisseur de couche de 250 µm à 700 µm, en particulier de 300 µm à 400 µm, préférablement de 350 µm.

9. Implant selon l'une quelconque des revendications précédentes, **caractérisé en ce que** le corps moulé de métal ou d'alliage est réalisé en forme de coquille ou en forme de segment de coquille sphérique.

10. Implant selon l'une quelconque des revendications précédentes, **caractérisé en ce que** le dispositif de croissance osseuse est réalisé au moins partiellement sur le corps moulé de métal ou d'alliage.

11. Implant selon l'une quelconque des revendications 1 à 5, **caractérisé en ce que** le dispositif de croissance osseuse est un corps moulé de métal ou d'alliage, en particulier un corps moulé de métal ou d'alliage en forme de coquille ou en forme de segment de coquille sphérique.

12. Implant selon la revendication 11, **caractérisé en ce que** le corps moulé de métal ou d'alliage présente une épaisseur de 0,2 mm à 2 mm, en particulier de 0,5 mm.

13. Implant selon l'une quelconque des revendications précédentes, **caractérisé en ce que** le dispositif de croissance osseuse présente un matériau contenant du calcium, préférablement un matériau de phosphate de calcium, en particulier choisi dans le groupe constitué par le phosphate monocalcique monohydraté (MCPM), le phosphate monocalcique anhydride (MCPA), le phosphate dicalcique anhydride (DCPA), le phosphate dicalcique dihydraté (DCPD), le phosphate octacalcique (OCP), le phosphate tricalcique α (α-TCP), le phosphate tricalcique β (β-TCP), le phosphate de calcium amorphe (ACP), l'hydroxyapatite (HA), l'hydroxyapatite déficiente en calcium (CdHA), l'hydroxyapatite substituée, l'hydroxyapatite non stœchiométrique, l'hydroxyapatite à l'échelle nanométrique, la fluoroapatite, la chloroapatite, la carbonate-fluor-apatite, le phosphate de calcium biphasique, le phosphate tétracalcique (TTCP), le sulfate de calcium (CaSO₄), le sulfate de calcium hémihydraté (CaSO₄ × 0,5 H₂O), le sulfate de calcium dihydraté (CaSO₄ × 2 H₂O), l'oxyde de calcium (CaO), l'hydroxyde de calcium (Ca(OH)₂), le carbonate de calcium (CaCO₃), le glycérophosphate de calcium, le citrate de calcium, le lactate de calcium, l'acétate de calcium, le tartrate de calcium, le chlorure de calcium (CaCl₂), le silicate de calcium et des mélanges correspondants.

14. Implant selon l'une quelconque des revendications 1 à 12, **caractérisé en ce que** le dispositif de croissance osseuse présente un métal ou un alliage, en particulier choisi dans le groupe constitué par le magnésium, le tantale, le titane, le nickel, un alliage de magnésium, un alliage de tantale, un alliage de titane, un alliage de chrome-cobalt, un alliage de chrome-cobalt-molybdène, un acier comme un acier inoxydable et des combinaisons correspondantes.
